# EUROPÄISCHE PATENTSCHRIFT

(11) **EP 0 693 180 B1**
(45) Veröffentlichungstag und Bekanntmachung des Hinweises auf die Patenterteilung: **29.03.2000**
(21) Anmeldenummer: 95901310.3
(22) Anmeldetag: 01.12.1994
(51) Int. Cl.: G01N 33/00, G01N 27/49

(54) **AMMONIAKSENSOR**
AMMONIA SENSOR
DETECTEUR D'AMMONIAC

(30) Priorität: 09.02.1994 CH 38494
(43) Veröffentlichungstag der Anmeldung: 24.01.1996
(73) Patentinhaber: Huggenberger, Christian, Dr., 8048 Zürich (CH)
(72) Erfinder: Huggenberger, Christian, Dr., 8048 Zürich (CH)
(74) Vertreter: Fiener, Josef
(86) Internationale Anmeldenummer: CH9400231
(87) Internationale Veröffentlichungsnummer: WO9522055

(56) Entgegenhaltungen:
- EP-A- 0 395 927
- EP-A- 0 496 527
- EP-A- 0 556 558
- GB-A- 2 225 859

## Beschreibung

Die Erfindung betrifft einen amperometrischen Ammoniak-Sensor gemäss dem Oberbegriff des Patentanspruches 1.

Zur Detektion von toxischen Gasen haben sich in vielen Anwendungen amperometrisch arbeitende Gassensoren bewährt, deren Wirkungsweise auf einer direkten elektrochemischen Oxidation oder Reduktion des zu messenden Gases beruht. Der durch Gasdiffusion aufrechterhaltene Zellenstrom ist eine Funktion der Gaskonzentration und erweist sich in guter Nährung als linear. Da sich Ammoniak elektrokatalytisch unter Normalbedingungen nur schwer oxidieren lässt, ist eine einfache Detektion auf diesem Wege nicht möglich.

Gemäss dem heutigen Stand der Technik sind amperometrische Ammoniaksensoren bekannt, die als Konzentrationszellen konzipiert sind (H. Komiya, Bionics Instrument Co., Ldt., The 1984 International Chemical Congress of Pacific Basin Societies): Messelektrode und Gegenelektrode bestehen aus Silber und befinden sich in einem Silbernitrat-Elektrolyten. Ammoniakgas gelangt durch eine gaspermeable Membran zur Messelektrode, wo folgende Reaktion stattfindet:

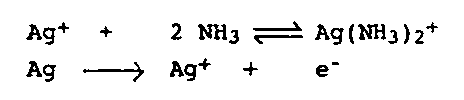

Der Nachteil besteht nun darin, dass sich bei Begasung mit Ammoniak die Silberelektrode ständig verbraucht. Zudem ist der Elektrolyt an der Luft instabil und zersetzt sich unter Bildung einer braunen Fällung. Infolge der begrenzten Lebensdauer müssen die Membran, der Elektrolyt und die Elektrode häufig ersetzt oder gereinigt werden.

Die Offenlegungsschrift DE 38 41 622 (Offenlegungstag 13.06.1990) beschreibt einen anderen amperometrischen Gassensor, der nach folgendem Prinzip arbeitet: Der Elektrolyt enthält ein gelöstes Ammoniumsalz, das in Gegenwart von Ammoniak zu einem leicht oxidierbaren Amin reagiert. Durch Anlegen eines Potentials zwischen den Elektroden wird das Amin zu einem Radikalkation oxidiert, das in Folgereaktionen involviert ist. Der Messstrom ist eine Funktion der Ammoniakkonzentration. Bei jeder Begasung wird allerdings der Elektrolyt irreversibel verbraucht. Zudem muss eine Bias-Spannung angelegt werden.

Aus der EP-A-0 395 927 ist eine Meßzelle zur Ammoniakbestimmung bekannt, in der die Meßelektrode mit einem festen Kobalt-Oxid-Überzug versehen ist. Diese Meßzelle weist eine hohe Langzeitstabilität auf, so daß auch größere Ammoniakmengen bestimmt werden können und sie keine Querempfindlichkeit gegenüber CO aufweisen soll.

Aus der EP-A-0 556 558 ist eine Weiterentwicklung dazu bekannt, wobei anstelle des Kobaltoxids ein Oxid der Platinmetallgruppe als Überzug der Meßelektrode verwendet wird. Die Langzeitstabilität und die Empfingdlichkeit werden dadurch erhöht und die Querempfindlichkeit verringert. Jedoch ist die Einstellung eines Biaspotentials erforderlich.

Die Aufgabe der Erfindung besteht darin, eine alternative Meßzelle zur Bestimmung von Ammoniak oder dessen Derivaten bereitzustellen.

Die Aufgabe wird mit den erfindungsgemässen Merkmalen nach dem kennzeichnenden Teil des Patentanspruches 1 gelöst. Vorteilhafte Weiterbildungen sind Gegenstand der Unteransprüche.

Der vorgeschlagene Sensor ist als amperometrischer Sensor konzipiert, der sowohl als 2-Elektroden- als auch Mehrelektrodenausführung ausgelegt werden werden kann. Als Elektrolyt dient ein Co(II)-Salz in einer wässrigen Lösung, die vorzugsweise einen hygroskopischen Zusatz enthält, um einem Verdunsten vorzubeugen, wobei das durch Luftsauerstoff in wässriger Lösung nicht oxidierbare Cobalt(II)-Ion mit Ammoniak ein komplexes durch Sauerstoff oxidierbares Ion bildet. Die Elektroden sind als Membranelektroden konzipiert, wobei der Membranträger eine Sperre für den Elektrolyten darstellt. Als Elektrodenmaterialien werden feinverteilte edelmetall- oder kohlenstoffhaltige Katalysatoren verwendet. Das durch die Messelektrode diffundierende Ammoniakgas geht an der Katalysatoroberfläche folgende Reaktion ein:

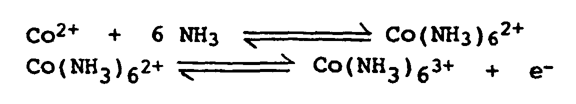

An der Gegenelektrode kommt folgende Reaktion in Gang:

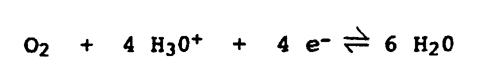

Da es sich um Gleichgewichtsreaktionen handelt, kehrt das System nach dem Absetzen der Begasung allmählich wieder in den Ausgangszustand zurück, wobei sich der Elektrolyt regeneriert. Zwischen den niederohmig verbundenen Elektroden fliesst infolge der Redox-Reaktion ein Strom, der eine Funktion der Ammoniakgaskonzentration ausserhalb des Sensors ist.

Die Erfindung ist unter anderem in den Zeichnungen erläutert. Es zeigen:
- Fig. 1a: Längsschnitt einer Membranelektrode;
- Fig. 1b: Membranelektrode, von unten gesehen;

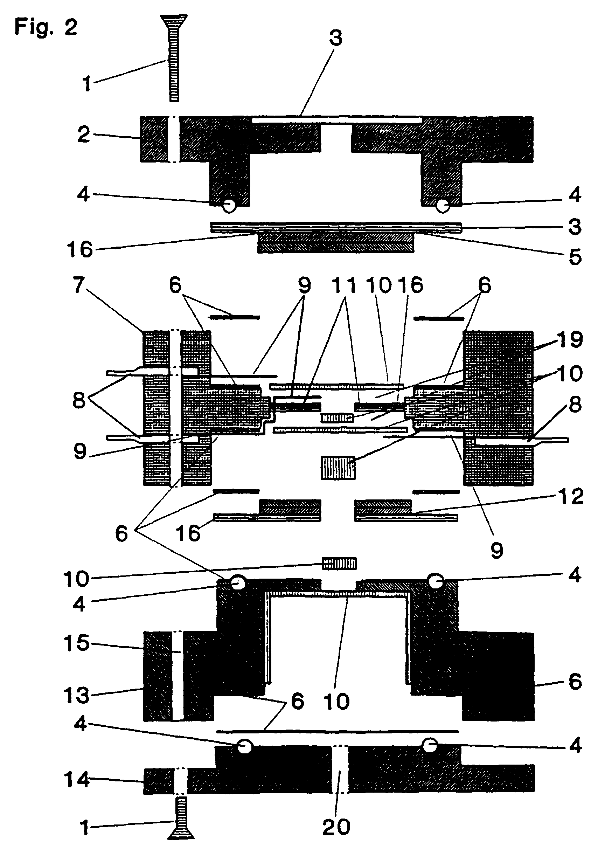

- Fig. 2: Längsschnitt eines Ammoniaksensors, mit folgenden Einzelteilen: Befestigungsschraube 1, säurebeständigem Kunststoff-O-Ring 4, ungesintertem PTFE-Band 6, aus Polycarbonat-Kunststoff gefertigtem Zentralkörper 7, Kontaktfeder 8, aus einer Folie der Stärke 0.025 mm geschnittenem Platinband 9, Tampon aus Glasfaser-Filter-Material 10, Körper für den Druckausgleich aus Polycarbonat-Kunststoff 13, Deckel aus Polycarbonat-Kunststoff mit einem Loch für den Druckausgleich 14, Bohrung mit einem Gewinde für eine Befestigungsschraube 15, Druckausgleichsöffnung mit einem Durchmesser von ca. 0.5 mm und einer Kapillarlänge von ca. 5 mm 20.

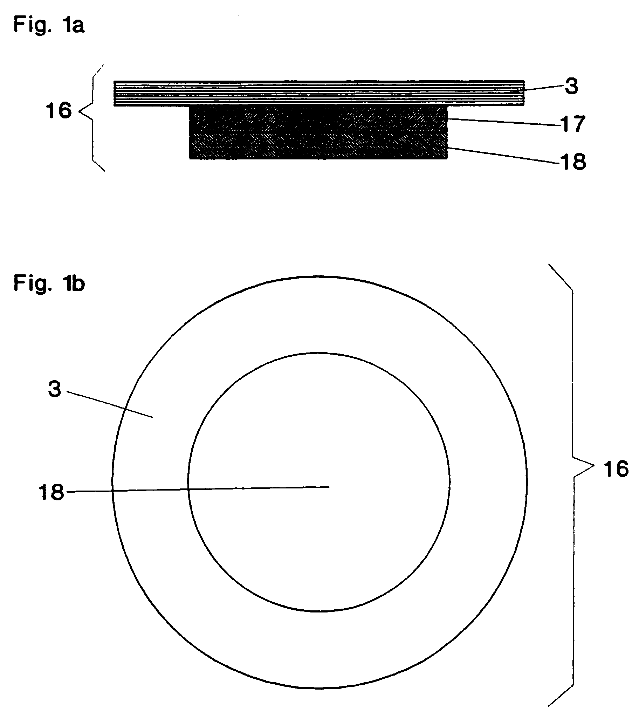

Die Elektroden 5, 11, 12 (Fig. 2) bestehen vorzugsweise aus einem flachen, porösen PTFE-(Polytetrafluorethylen)-Membran-Träger 3, der mit einer porösen gesinterten Schicht versehen ist, die ein Gemisch von feinverteiltem Katalysator- und PTFE-Pulver enthält. Der Membranträger stellt einerseits eine Sperre für den Elektrolyten dar, andererseits ist er für das Messgas permeabel. Die Katalysatorschicht steht in Kontakt zum Elektrolyten und wird von diesem penetriert. Für die Messelektrode 5 wird als Katalysator vorzugsweise Gold, für Referenz- und Gegenelektroden 11 bzw. 12 vorzugsweise Grafit verwendet, da in diesem Fall die Signalempfindlichkeit, Signalstabilität und Ansprechzeit besonders gut sind und eine Querempfindlichkeit auf Kohlenmonoxid unterbleibt. Aufgrund der Potentialdifferenz zwischen unterschiedlichen Elektroden entsteht allerdings im Leerbetrieb an der Umgebungsluft ein angehobener Nullstrom, der temperaturabhängig ist. Dieser Effekt kann vorteilhaft unter Beibehaltung der übrigen guten Eigenschaften dadurch kompensiert werden, dass gemäss den Figuren 1a und 1b der Elektrodenaufbau für sämtliche Elektroden, mindestens aber für Mess- und Referenzelektrode, identisch konzipiert wird: 3 ist eine poröse PTFE-Membran, 17 ist eine erste poröse gesinterte Katalysatorschicht aus einem Gemisch von feinverteiltem Gold- und PTFE-Pulver und 18 ist eine zweite poröse gesinterte Katalysatorschicht aus feinverteiltem Grafit- und PTFE-Pulver. Durch die Gegenreaktion an der 2. Schicht 18 der Messelektrode 5 geht zwar ein Anteil des erfassbaren Messstromes verloren, aber bei genügend kleinem Innenwiderstand zwischen Mess- und Referenzelektrode 5 bzw. 11 ist das Signal immer noch kräftig.

In einer ersten Ausbildungsart gemäss Fig. 2 wurde eine Messelektrode 5 aus Gold und eine Referenz- bzw. Gegenelektrode 11 bzw. 12 aus Grafit eingesetzt. Der gasseitige Deckel 2 war mit 4 Löchern von einem Durchmesser von je 2 mm und einer Länge von je 2 mm versehen. Zur Herstellung der Membranelektroden 16 wurden Gemische 17 von feinverteiltem Gold- und PTFE-Pulver bzw. Gemische 18 von feinverteiltem Grafit- und PTFE-Pulver auf poröse PTFE-Membranen 3 beschichtet und in einem Wärmeschrank bei 360 °C gesintert. Als hygroskopischer Zusatz für den Elektrolyten wurde 1,2-Ethandiol (Ethylenglycol) verwendet. Als Co²⁺-Salz wurde CoCl₂.6 H₂0 gewählt. Die Konzentration von CoCl₂.6 H₂0 in einem Gemisch von 20 Mass.-% Wasser und 80 Mass.-% Ethylenglycol war 0.01 molal. Rückseitig wurde soviel Elektrolyt in die Elektrolytkammer 19 eingefült, bis der Glasfaser-Tampon 10 gesättigt war. Nach einer Woche bei kurzgeschlossener Mess- und Referenzelektrode hatte sich der Nullstrom zwischen den Elektroden 5, 11 auf 0.5 µA stabilisiert. Beim Begasen mit 150 ppm NH₃ stieg der Strom zwischen Mess- und Referenzelektrode 5, 11 innert weniger Minuten auf ein stabiles, reproduzierbares Plateau von 3.7 µA. Nach dem Absetzen der Begasung sinkt der Strom innert weniger Minuten wieder auf den Ausgangswert des Nullstromes. Beim Begasen mit 200 ppm Kohlenmonoxid wurde keine Querempfindlichkeit festgestellt.

In einer zweiten Ausbildungsart gemäss Fig. 2 wurden als Mess- und Referenzelektrode 5 bzw. 11 Membranelektroden 16 gemäss den Figuren 1a und 1b eingesetzt. Der gasseitige Deckel 2 war mit 4 Löchern von einem Durchmesser von je 1.5 mm und einer Länge von je 2 mm versehen. Die Gegenelektrode 12 war die gleiche wie in der ersten Ausbildungsart. Die Herstellung der zweischichtigen Membranelektroden 16 erfolgte folgendermassen: In einem ersten Schritt wurde ein Gemisch 17 von feinverteiltem Gold- und PTFE-Pulver auf eine poröse PTFE-Membran 3 beschichtet. In einem zweiten Schritt wurde auf diese Schicht 17 ein Gemisch 18 von feinverteiltem Grafit- und PTFE-Pulver beschichtet. Die Membran 3 wurde anschliessend in einem Wärmeschrank bei 360 °C gesintert. Als hygroskopischer Zusatz diente ebenfalls Ethylenglycol. Ebenso wurde CoCl₂.6 H₂0 verwendet. Die Konzentration in einem Gemisch von 20 Mass.-% Wasser und 80 Mass.-% Ethylenglycol war diesmal 0.1 molal. Die Elektrolytkammer 19 wurde auf die gleiche Weise befüllt. Der Nullstrom zwischen Mess- und Referenzelektrode hatte sich bereits nach einem Tag auf 0.02 µA stabilisiert. Beim Begasen mit 150 ppm NH₃ stieg der Strom zwischen Mess- und Referenzelektrode 5 bzw. 11 innert weniger Minuten, aber deutlich schneller als in der ersten Ausbildungsart auf ein stabiles, reproduzierbares Plateau von 1.0 µA. Nach dem Absetzen der Begasung sinkt der Messstrom erheblich schneller als in der ersten Ausbildungsart wiederum auf den Ausgangswert des Nullstromes. Beim Begasen mit 200 ppm Kohlenmonoxid wurde ebenfalls keine Querempfindlichkeit festgestellt.

Beide Ausbildungsarten zeigen eine gute lineare Abhängigkeit des Messstromes von der Ammoniakkonzentration.

Der beschriebene Ammoniaksensor, der in einer kleinen, kompakten Bauweise hergestellt werden kann, eignet sich besonders für die Ueberwachung von Ammoniak oder dessen Derivaten in der Umgebungsluft, kann aber auch für die Bestimmung von Ammoniak in flüssigen Messproben eingesetzt werden. Der Sensor kann einer langen Ammoniakgas-Exposition ausgesetzt werden und detektiert auch höhere Ammoniakgas-Konzentrationen. Er besitzt keine Querempfindlichkeit gegenüber Kohlenmonoxid. Der Sensor hält sich selber in Betrieb und benötigt keine Bias-Spannung.

## Patentansprüche

1. Ammoniaksensor auf amperometrischer Basis, der mindestens zwei Elektroden (5,12) in einer mit einem Elektrolyten gefüllten Elektrolytkammer (19) umfasst, die zur Messprobe hin durch eine für Ammoniak permeable Membran (3) abgeschlossen ist,
dadurch gekennzeichnet,
dass der Elektrolyt ein von Luftsauerstoff in wässriger Lösung nicht oxidierbares Metall-Ion enthält, das mit Ammoniak ein komplexes, mit Sauerstoff oxidierbares Metall-Ion bildet.

2. Ammoniaksensor nach Anspruch 1, dadurch gekennzeichnet, dass der Elektrolyt ein Co(II)-Salz in wässriger Lösung umfasst.

3. Ammoniaksensor nach Anspruch 1 oder 2, dadurch gekennzeichnet, dass als Cobaltsalz Cobaltdichlorid verwendet wird.

4. Ammoniaksensor nach einem der Ansprüche 1 - 3, dadurch gekennzeichnet, dass der Elektrolyt einen hygroskopischen Zusatz enthält.

5. Ammoniaksensor nach einem der Ansprüche 1 - 4, dadurch gekennzeichnet, dass der hygroskopische Zusatz 1,2-Ethandiol umfasst.

6. Ammoniaksensor nach einem der Ansprüche 1 - 5, dadurch gekennzeichnet, dass der Anteil des hygroskopischen Zusatzes am Elektrolyten zwischen 1 und 99 Mass.-% beträgt.

7. Ammoniaksensor nach einem der Ansprüche 1 - 6, dadurch gekennzeichnet, dass der Anteil des hygroskopischen Zusatzes zwischen 70 und 90 Mass.-% beträgt.

8. Ammoniaksensor nach einem der Ansprüche 1 - 7, dadurch gekennzeichnet, dass die Konzentration des Cobaltsalzes im Elektrolyten im Bereich 0,001 - 1 molal liegt.

9. Ammoniaksensor nach einem der Ansprüche 1 - 8, dadurch gekennzeichnet, dass die Konzentration des Cobaltsalzes im Elektrolyten im Bereich 0.01 - 0,1 molal liegt.

10. Ammoniaksensor nach einem der Ansprüche 1 - 9, dadurch gekennzeichnet, dass zusätzlich eine Referenzelektrode (11) vorgesehen ist.

11. Ammoniaksensor nach einem der Ansprüche 1 - 10, dadurch gekennzeichnet, dass die Messelektrode (5) aus Gold und die Referenz- bzw. Gegenelektrode (11 bzw. 12) aus Grafit bestehen.

12. Ammoniaksensor nach einem der Ansprüche 1 - 11, dadurch gekennzeichnet, dass jeweils eine PTFE-Membran (3) für die Messelektrode (5) mit einem Gemisch (17) aus feinverteiltem Gold- und PTFE-Pulver bzw. für die Referenz- bzw. Gegenelektrode (11 bzw. 12) mit einem Gemisch (18) aus feinverteiltem Grafit- und PTFE-Pulver beschichtet und anschliessend in einem Wärmeschrank gesintert wird.

13. Ammoniaksensor nach einem der Ansprüche 1 - 11, dadurch gekennzeichnet, dass jeweils eine PTFE-Membran (3) für die Messelektrode (5) sowie die Referenzelektrode (11) mit einem Gemisch (17) aus feinverteiltem Gold- und PTFE-Pulver, sodann mit einem Gemisch aus feinverteiltem Grafit- und PTFE-Pulver (18) beschichtet und anschließend in einem Wärmeschrank gesintert wird.

14. Ammoniaksensor nach einem der Ansprüche 1 - 13, dadurch gekennzeichnet, dass die Elektrolytkammer (19) gasseitig mit einer für Ammoniak durchlässigen Membran (3), radial von einem zylindrischen Zentralkörper (7) sowie rückseitig von einem Druckausgleichskörper (13) mit einem mit einer Druckausgleichsöffnung (20) versehenen Deckel (14) begrenzt ist.

15. Ammoniaksensor nach einem der Ansprüche 1 - 14, dadurch gekennzeichnet, dass Kontaktfedern (8), Platinbänder (9) und mit Elektrolyt getränkte Glasfaser-Tampons (10) zwecks Kontaktierung der Elektroden (5,11,12), O-Ringe (4) und Schrauben (1) für entsprechende Gewindebohrungen (15) zwecks Verschliessung der Elektrolytkammer (19) sowie Abschnitte (6) aus ungesintertem PTFE-Band zwecks Abdichtung der Elektrolytkammer (19) vorgesehen sind.

## Claims

1. Ammonia sensor based on amperometry, comprising at least two electrodes (5, 12) accommodated in an electrolyte chamber (19) which is filled with an electrolyte and sealed relative to the measuring sample by a membrane (3) which is permeable to ammonia,
characterized in that
the electrolyte contains a metal ion which is non-oxidizable by air oxygen in an aqueous solution and, with ammonia, forms a complex metal ion which is oxidizable with oxygen.

2. Ammonia sensor according to claim 1, characterized in that the electrolyte comprises a Co(II) salt in an aqueous solution.

3. Ammonia sensor according to either claim 1 or claim 2, characterized in that cobalt dichloride is used as the cobalt salt.

4. Ammonia sensor according to any of claims 1 to 3, characterized in that the electrolyte includes a hygroscopic additive.

5. Ammonia sensor according to any of claims 1 to 4, characterized in that the hygroscopic additive comprises 1,2-ethane-diol.

6. Ammonia sensor according to any of claims 1 to 5, characterized in that the percentage of the hygroscopic additive in the electrolyte is in a range of from 1 to 99 percent by weight.

7. Ammonia sensor according to any of claims 1 to 6, characterized in that the percentage of the hygroscopic additive is in a range of from 70 to 90 percent by weight.

8. Ammonia sensor according to any of claims 1 to 7, characterized in that the concentration of the cobalt salt in the electrolyte is in a range of from 0.001 to 1 molal.

9. Ammonia sensor according to any of claims 1 to 8, characterized in that the concentration of the cobalt salt in the electrolyte is in a range of from 0.01 to 0.1 molal.

10. Ammonia sensor according to any of claims 1 to 9, characterized in that there is further provided a reference electrode (11).

11. Ammonia sensor according to any of claims 1 to 10, characterized in that the measuring electrode (5) is made of gold and the reference and counter electrodes (11 and 12), respectively, are made of graphite.

12. Ammonia sensor according to any of claims 1 to 11, characterized in that one PTFE membrane (3) for the measuring electrode (5) is coated with a mixture (17) consisting of finely distributed gold and PTFE powders, and one PTFE membrane (3) for the reference and counter electrodes (11 and 12), respectively, is coated with a mixture (18) consisting of finely distributed graphite and PTFE powders, and subsequently, each of said membranes (3) is sintered in a warming box.

13. Ammonia sensor according to any of claims 1 to 11, characterized in that one PTFE membrane (3) each for the measuring electrode (5) and the reference electrode (11) is primed with a mixture (17) of finely distributed gold and PTFE powders, then coated with a mixture (18) of finely distributed graphite and PTFE powders, and subsequently sintered in a warming box.

14. Ammonia sensor according to any of claims 1 to 13, characterized in that the electrolyte chamber (19) is limited, to the gas side, by a membrane (3) permeable to ammonia, radially, by a cylindrical central body (7), and to the rear, by a pressure-compensating body (13) comprising a cap (14) which includes a breather (20).

15. Ammonia sensor according to any of claims 1 to 14, characterized in that there are provided contact springs (8), platinum tapes (9), and fibreglass swabs (10) saturated with electrolyte for the purpose of contacting the electrodes (5, 11, 12), O-rings (4) and screws (1) for corresponding tapholes (15) for the purpose of locking up the electrolyte chamber (19), and segments (6) of non-sintered PTFE tape for the purpose of sealing the electrolyte chamber (19).

## Revendications

1. Détecteur d'ammoniac de type ampérométrique, qui comporte au moins deux électrodes (5, 12) dans une chambre électrolytique (19) remplie d'électrolyte, laquelle chambre est séparée de la sonde de mesure par une membrane perméable à l'ammoniac (3), caractérisé en ce que l'électrolyte contient un ion métallique non oxydable par l'oxygène de l'air en solution aqueuse, et qui forme avec l'ammoniac un ion métallique complexe oxydable par l'oxygène.

2. Détecteur d'ammoniac selon la revendication 1, caractérisé en ce que l'électrolyte comporte un sel de Co(II) en solution aqueuse.

3. Détecteur d'ammoniac selon la revendication 1 ou 2, caractérisé en ce que l'on utilise comme sel de cobalt du dichlorure de cobalt.

4. Détecteur d'ammoniac selon l'une des revendications 1 à 3, caractérisé en ce que l'électrolyte comporte un additif hygroscopique.

5. Détecteur d'ammoniac selon l'une des revendications 1 à 4, caractérisé en ce que l'additif hygroscopique comporte du 1,2-éthanediol.

6. Détecteur d'ammoniac selon l'une des revendications 1 à 5, caractérisé en ce que la proportion de l'additif hygroscopique dans l'électrolyte est comprise entre 1 et 99 % en masse.

7. Détecteur d'ammoniac selon l'une des revendications 1 à 6, caractérisé en ce que la proportion de l'additif hygroscopique dans l'électrolyte est comprise entre 70 et 90 % en masse.

8. Détecteur d'ammoniac selon l'une des revendications 1 à 7, caractérisé en ce que la concentration du sel de cobalt dans l'électrolyte est comprise dans la gamme allant de 0001 à 1 molale.

9. Détecteur d'ammoniac selon l'une des revendications 1 à 8, caractérisé en ce que la concentration du sel de cobalt dans l'électrolyte est comprise dans la gamme allant de 0,01 à 0,1 molale.

10. Détecteur d'ammoniac selon l'une des revendications 1 à 9, caractérisé en ce qu'est prévue en outre une électrode de référence (11).

11. Détecteur d'ammoniac selon l'une des revendications 1 à 10, caractérisé en ce que l'électrode de mesure (5) se compose d'or et l'électrode de référence ou la contre-électrode (11 ou 12) se compose de graphite.

12. Détecteur d'ammoniac selon l'une des revendications 1 à 11, caractérisé en ce qu'une membrane de PTFE (3) est revêtue respectivement pour l'électrode de mesure (5) d'un mélange (17) composé de poudre finement dispersée d'or et de PTFE, ou pour l'électrode de référence ou la contre-électrode (11 ou 12) d'un mélange (18) composé de poudre finement dispersée de graphite et de PTFE, puis est frittée dans une étuve.

13. Détecteur d'ammoniac selon l'une des revendications 1 à 11, caractérisé en ce qu'une membrane de PTFE (3) respectivement pour l'électrode de mesure (5) et pour l'électrode de référence (11) est revêtue d'un mélange (17) composé de poudre finement dispersée d'or et de PTFE, puis d'un mélange (18) composé de poudre finement dispersée de graphite et de PTFE, puis est frittée dans une étuve.

14. Détecteur d'ammoniac selon l'une des revendications 1 à 13, caractérisé en ce que la chambre électrolytique (19) est séparée du gaz par une membrane perméable à l'ammoniac (3), séparée radialement du corps central cylindrique (7) et vers l'arrière du corps d'égalisation de pression (13) par un couvercle (14) équipé d'une ouverture d'égalisation de pression (20).

15. Détecteur d'ammoniac selon l'une des revendications 1 à 14, caractérisé en ce que sont prévus des ressorts de contact (8), des lames de platine (9) et des tampons de fibres de verre imprégnés d'électrolyte (10) en vue d'établir le contact des électrodes (5, 11, 12), des joints toriques (4) et des vis (1) d'alésages filetés correspondants (15) en vue de fermer la chambre électrolytique (19), ainsi que des segments (6) composés de bandes de PTFE non frittées en vue de rendre étanche la chambre électrolytique (19).
